# EUROPÄISCHE PATENTSCHRIFT

(11) **EP 3 351 220 B1**
(45) Veröffentlichungstag und Bekanntmachung des Hinweises auf die Patenterteilung: **29.04.2020**
(21) Anmeldenummer: 17182056.6
(22) Anmeldetag: 19.07.2017
(51) Int. Cl.: A61F 5/01, A61B 17/64, A61F 13/08

(54) **KOMPRESSIONSTEXTIL ZUR POSTOPERATIVEN KOMPRESSIONSBEHANDLUNG EINER KÖRPEREXTREMITÄT**
COMPRESSION TEXTILE FOR POSTSURGICAL COMPRESSION TREATMENT OF A PHYSICAL EXTREMITY
TISSU DE COMPRESSION DESTINÉ AU TRAITEMENT POSTOPÉRATIF PAR COMPRESSION D'UNE EXTRÉMITÉ DU CORPS

(30) Priorität: 20.01.2017 DE 102017101042
(43) Veröffentlichungstag der Anmeldung: 25.07.2018
(73) Patentinhaber: Julius Zorn GmbH, 86551 Aichach (DE)
(72) Erfinder: Schettler, Uwe, 86551 Aichach (DE)
(74) Vertreter: Charrier Rapp & Liebau

(56) Entgegenhaltungen:
- EP-A2- 2 138 140
- WO-A1-99/36019
- DE-A1-102013 019 490
- US-A1- 2013 283 500

## Beschreibung

Die Erfindung betrifft ein System zur postoperativen Kompressionsbehandlung einer Körperextremität bestehend aus einem Kompressionstextil und einem externen Fixateur mit einer Mehrzahl von Knochenstiften, die von außen in einem Knochen der Körperextremität befestigt worden sind.

Zur Behandlung von Knochenbrüchen, insbesondere bei offenen Frakturen oder Trümmerfrakturen, werden externe Fixateure (auch als "Fixateur externe" bezeichnet) eingesetzt, die ein von außen am betroffenen Knochen befestigtes Haltesystem umfassen, das dazu dient, die gebrochenen Knochenteile während des Heilungsprozesses an der Bruchstelle in der richtigen Knochengeometrie zu halten und ruhig zu stellen. Der externe Fixateur umfasst dabei eine Mehrzahl von Knochenstiften, die von außen (durch die Haut und das den Knochen umgebende Körpergewebe) eingebracht und in den Knochenteilen befestigt werden. Die in den Knochenteilen befestigten Knochenstifte werden außerhalb mit einer starren Haltevorrichtung, die beispielsweise Metallstangen umfasst, fest verbunden, um dadurch eine Fixierung der Knochenteile sowie eine Ruhigstellung des betroffenen Körperteils zu gewährleisten. Eine solche Vorrichtung zur äußeren Fixierung gebrochener Knochen ist beispielsweise aus der DE 32 29 313 A1 bekannt.

Aufgrund der für den Heilungsprozess bei Knochenbrüchen erforderlichen Ruhigstellung des betroffenen Körperteils besteht insbesondere bei Knochenbrüchen im Fuß- und Beinbereich die Gefahr, dass sich Thrombosen bilden. Die Bildung einer venösen Thrombose kann bei bettlägerigen Patienten durch eine Kompressionsbehandlung mit einem Thromboseprophylaxestrumpf verhindert werden. Bei einem Thromboseprophylaxestrumpf handelt sich um einen Kompressionsstrumpf mit einer im Vergleich zu medizinischen Kompressionsstrümpfen geringeren Kompressionswirkung (der von einem Thromboseprophylaxestrumpf auf das Bein ausgeübten Kompressionsdruck liegt dabei regelmäßig unterhalb der Kompressionsklasse I von medizinischen Kompressionsstrümpfen). Des Weiteren werden zur Verbesserung des Heilungsverlaufs und zur Stabilisierung des betroffenen Körperteils bei Knochenbrüchen Kompressionstextilien, beispielsweise in der Form von medizinischen Kompressionsstrümpfen oder Kompressionsärmeln, eingesetzt, um bspw. postoperative (Lymph-)Ödeme zu verhindern oder zu reduzieren und Schwellungen und Ergüsse zu verringern. Das Kompressionstextil bewirkt dabei durch den auf die Körperextremität wirkenden Kompressionsdruck eine Ruhigstellung der Körperextremität, eine Verbesserung der Blutzirkulation und eine Erhöhung des Gewebedrucks. Insbesondere wenn es sich bei dem Kompressionstextil um ein Kompressionsgestrick mit einem von distal nach proximal abnehmenden Kompressionsdruck handelt, kann der Blutrückfluss in den Venen durch den das Kompressionstextil unterstützt und verbessert werden. Des Weiteren fördert eine postoperative Kompressionsbehandlung den Abfluss von Lymphflüssigkeit und durch die Erhöhung des Gewebedrucks eine Rückresorption von postoperativen oder posttraumatischen Ödemen, was ebenfalls positiv zum Heilungsverlauf bei Frakturen einer Körperextremität beiträgt.

Aus der US-amerikanischen Patentanmeldung US 2013/0283500 A1 geht ein Kompressionstextil mit einem ersten Abschnitt, der sich zumindest teilweise um ein Körperteil wickelt, um eine Kompression für das Körperteil bereitzustellen, und einem zweiten Abschnitt, der sich zumindest teilweise um das Körperteil wickelt, hervor, wobei der zweite Abschnitt auf den ersten Abschnitt gewickelt ist oder an dem ersten Abschnitt befestigt ist.

Eine Kompressionsbandage bestehend aus einem elastischen Material sowie Befestigungen zur lösbaren Anbringung des elastischen Materials in einem gestreckten Zustand um das Bein eines Patienten herum, ist aus der WO 99/36019 A1 bekannt.

Wenn zur Behandlung einer Fraktur im Bereich einer Körperextremität, beispielsweise einem Bein oder einem Arm, ein externer Fixateur zur äußeren Fixierung der gebrochenen Knochen eingesetzt wird, können die herkömmlich verwendeten Kompressionstextilien, die in der Regel strumpf- bzw. schlauchförmig ausgebildet sind, nicht eingesetzt werden, da das strumpf- bzw. schlauchförmige Kompressionstextil wegen der aus der Körperextremität herausragenden Knochenstifte des Fixateurs nicht über die Körperextremität gezogen werden kann.

Hiervon ausgehend liegt der Erfindung die Aufgabe zugrunde, ein System zur postoperativen Kompressionsbehandlung einer Körperextremität bei Frakturen bereit zu stellen, wobei das System aus einem Kompressionstextil und einem externen Fixateur, der zur Fixierung der gebrochenen Knochen eingesetzt werden kann, besteht. Das Kompressionstextil soll dabei die Ausübung eines variablen und individuell einstellbaren Kompressionsdrucks auf die Körperextremität ermöglichen. Weiterhin soll ein einfaches Anlegen und Abnehmen des Kompressionstextils an der Körperextremität trotz des dort angeordneten Fixateurs möglich sein.

Diese Aufgaben werden mit einem System mit den Merkmalen des Anspruchs 1 gelöst. Bevorzugte Ausführungsformen des erfindungsgemäßen Systems sind den abhängigen Ansprüchen zu entnehmen.

Das erfindungsgemäße System zur postoperativen Kompressionsbehandlung einer Körperextremität besteht aus einem Kompressionstextil und einem externen Fixateur, wobei der externe Fixateur eine Mehrzahl von Knochenstiften umfasst, die von außen in einem Knochen der Körperextremität befestigt worden sind. Das Kompressionstextil umfasst dabei einen sich längs einer Längsrichtung erstreckenden Kompressionsabschnitt aus einem Kompressionsgestrick mit einem ersten und einem zweiten seitlichen Randabschnitt, sowie mindestens eine erste Gruppe von Befestigungslaschen mit mehreren Befestigungslaschen aus einem elastischen Textilmaterial, welche jeweils an einem der seitlichen Randabschnitte des Kompressionsabschnitts angeordnet sind und über Befestigungsmittel verfügen, wobei benachbarte Befestigungslaschen der ersten Gruppe von Befestigungslaschen jeweils durch Einschnitte voneinander getrennt sind.

Optional kann bei dem Kompressionstextil eine zweite Gruppe von Befestigungslaschen vorgesehen sein, welche ebenfalls mehrere Befestigungslaschen aus einem elastischen Textilmaterial umfasst, die an dem anderen seitlichen Randabschnitt des Kompressionsabschnitts angeordnet sind. Mittels der Befestigungsmittel, die beispielsweise an den freien Enden der Befestigungslaschen der ersten Gruppe von Befestigungslaschen angeordnet sein können, sind die Befestigungslaschen der ersten Gruppe an dem Ihnen gegenüberliegenden Randabschnitt des Kompressionsabschnitts oder an den gegenüberliegenden Befestigungslaschen der zweiten Gruppe befestigbar.

Das Kompressionstextil kann dadurch an der Körperextremität angelegt und dort fixiert werden, indem die Befestigungslaschen der ersten Gruppe und - sofern vorhanden - auch die

Befestigungslaschen der zweiten Gruppe um die Körperextremität geschlungen und dort unter Zugspannung mit den ersten (und ggf. den optionalen zweiten) Befestigungsmitteln fixiert werden.

Die am betroffenen Knochen der Körperextremität befestigten Knochenstifte des Fixateurs können beim Anlegen des Kompressionstextils durch die Einschnitte zwischen benachbarten Befestigungslaschen der ersten Gruppe von Befestigungslaschen und - sofern vorhanden - der zweiten Gruppe von Befestigungslaschen hindurchgeführt werden. Beim Anlegen des Kompressionstextils an eine Körperextremität werden die Befestigungslaschen um die aus der Körperextremität ragenden Knochenstifte herumgelegt. Dadurch ist es möglich, trotz der aus der Körperextremität herausragenden Knochenstifte ein Kompressionstextil an der Körperextremität anzulegen.

Die Zugspannung, die beim Anlegen des Kompressionstextils auf die aus einem elastischen Textilmaterial gebildeten Befestigungslaschen ausgeübt wird, wird durch die Befestigung der Befestigungslaschen mittels der Befestigungsmittel fixiert. Daher üben die Befestigungslaschen der ersten Gruppe und ggf. der zweiten Gruppe von Befestigungslaschen in Verbindung mit dem Kompressionsgestrick des Kompressionsabschnitts einen Kompressionsdruck auf die darunter liegende Körperextremität aus. Die Höhe des Kompressionsdrucks kann dabei durch die auf die Befestigungslaschen ausgeübte Zugspannung sowie die Positionierung der freien Enden der Befestigungslaschen beim Anlegen des Kompressionstextils individuell eingestellt werden.

Dadurch ist es möglich, den auf die Körperextremität ausgeübten Kompressionsdruck bei dem Kompressionstextil individuell an die Behandlung des Patienten anzupassen. Weiterhin ist es möglich, den Kompressionsdruck auch während der Behandlungsdauer zu variieren und dem Behandlungsverlauf anzupassen. Durch den vom Kompressionstextil auf die Körperextremität ausgeübten Kompressionsdruck wird einerseits die Bildung von Thrombosen bei bettlägerigen Patienten verhindert und andererseits wird die Heilung der Fraktur durch eine Verbesserung der Blutzirkulation und des Abflusses von Lymphflüssigkeit gefördert. Darüber hinaus wird durch die Kompressionswirkung des Kompressionstextils der Gewebedruck in der Körperextremität erhöht, wodurch die Resorption von postoperativen oder posttraumatischen Ödemen beschleunigt wird.

Bevorzugt ist der Kompressionsabschnitt des Kompressionstextils aus einem Kompressionsgestrick gefertigt, dessen Dehnbarkeit quer zur Längsrichtung von proximal nach distal abnimmt, so dass der vom Kompressionsgestrick auf die Körperextremität ausgeübte Druck bei angelegtem Kompressionstextil von proximal nach distal zunimmt. Dadurch kann der venöse Blutfluss beschleunigt und der Abfluss von Lymphflüssigkeit verbessert werden.

Ein einfaches und schnelles Anlegen und Abnehmen des Kompressionstextils wird durch eine Ausbildung der Befestigungsmittel als Klettverschlüsse ermöglicht, mit denen die Befestigungslaschen unter einer Zugspannung und lösbar fixiert werden können. Besonders zweckmäßig ist es dabei, wenn die Klettverschlüsse, insbesondere die Hakenteile (Widerhäkchen) der Klettverschlüsse, abnehmbar an den Befestigungslaschen angeordnet sind. Dadurch ist es möglich, die Befestigungslaschen mittels der Befestigungsmittel an unterschiedlichen Stellen des Kompressionstextils zu fixieren, wodurch ein Durchführen der Knochenstifte durch die Einschnitte zwischen benachbarten Befestigungslaschen beim Anlegen des Kompressionstextils erleichtert wird.

Diese und weitere Vorteile sowie Merkmale der Erfindung ergeben sich aus dem nachfolgend unter Bezugnahme auf die begleitenden Zeichnungen näher beschriebenen Ausführungsbeispiel. Die Zeichnungen zeigen:
- **Figur** 1:: Perspektivische Darstellung eines an einem Unterschenkel eines Patienten angelegten Kompressionstextils zur postoperativen Kompressionsbehandlung in einem ersten Ausführungsbeispiel, zusammen mit einem am Unterschenkel befestigten externen Fixateur zur äußeren Fixierung gebrochener Knochen des Unterschenkels;
- **Figur 2:**: Schematische Darstellung eines zweiten Ausführungsbeispiels eines Kompressionstextils in einem geöffneten Zustand am Unterschenkel eines Patienten in einer Vorderansicht;
- **Figur 3:**: Schematische Darstellung des Kompressionstextils von Figur 2 am Unterschenkel eines Patienten in einer Rückansicht;

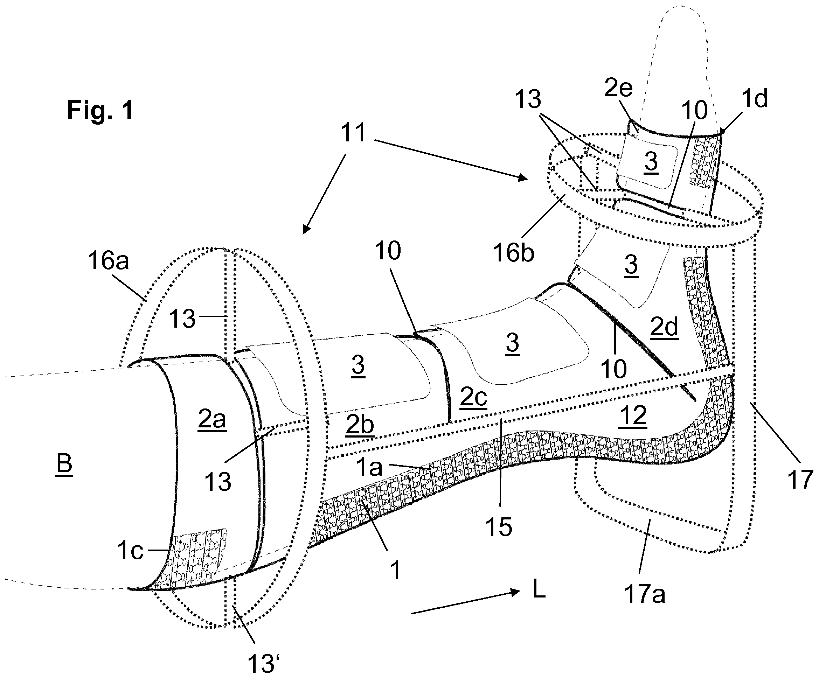

In Figur 1 ist ein Kompressionstextil zur postoperativen Kompressionsbehandlung einer Körperextremität in einem ersten Ausführungsbeispiel zusammen mit einem an der Körperextremität B befestigten externen Fixateur 11 gezeigt. Bei dem in Figur 1 dargestellten Ausführungsbeispiel handelt es sich bei der Körperextremität B um einen Unterschenkel, an dem zur Fixierung gebrochener Knochen ein externer Fixateur 11 mit einer Mehrzahl von Knochenstiften 13 angeordnet ist, wobei die Knochenstifte 13 von außen in die Knochenteile eines gebrochenen Knochens des Unterschenkels befestigt worden sind. Der externe Fixateur 11 umfasst neben den Knochenstiften 13, die im Bereich des Unterschenkels sowie im Bereich des Mittelfußes senkrecht aus der Körperextremität herausragen, äußere Fixierungselemente 15, 16, welche an den aus der Körperextremität herausragenden Knochenstiften 13 befestigt sind. Die Fixierungselemente 15, 16 umfassen in dem gezeigten Ausführungsbeispiel zwei Ringe 16, nämlich einen proximalen Ring 16a, der den Unterschenkel koaxial umgibt, und einen distalen Ring 16b, der am Fuß F angelegt ist und diesen umgreift. Die beiden Ringe 16 sind jeweils mit den herausragenden Knochenstiften 13 verbunden. An dem distalen Ring 16b ist ein Lagerungsbügel 17 angeordnet, der zur Lagerung der Körperextremität B dient und einen flachen Abschnitt 17a aufweist, der auf einer ebenen Unterlage, bspw. einer Matratze, aufgestellt werden kann. Der Lagerbügel 17 ist über zwei parallel in Längsrichtung der Körperextremität B und im Abstand zueinander verlaufende Befestigungsstangen 15 mit dem proximalen Ring 16a verbunden. Der externe Fixateur 11 hält gebrochene Knochenteile des Unterschenkels in der richtigen Knochengeometrie und stellt den Unterschenkel ruhig. Gleichzeitig wird eine stabile Lagerung auf dem Lagerbügel 17 ermöglicht.

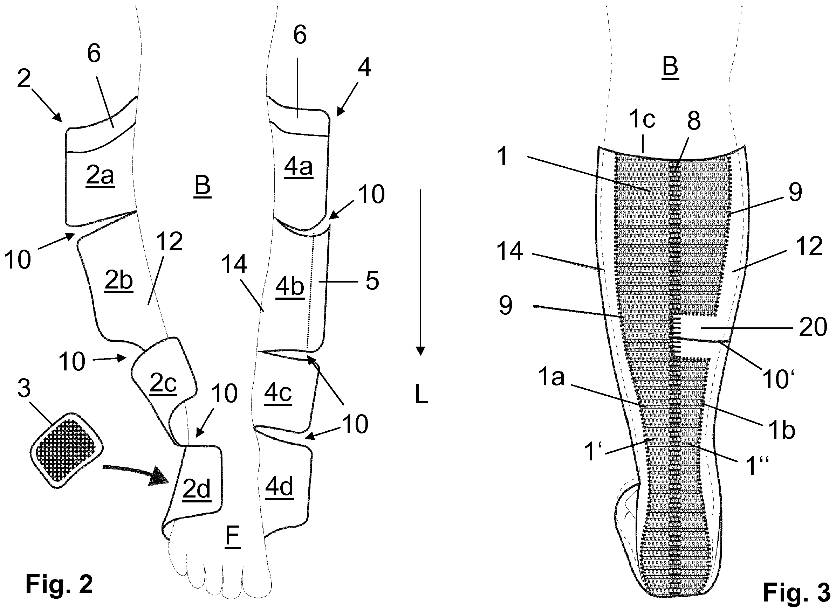

Zur Kompressionsbehandlung des Unterschenkels ist neben dem externen Fixateur 11 ein Kompressionstextil am Unterschenkel angelegt. Ein Ausführungsbeispiel des Kompressionstextils ist in den Figuren 2 und 3 im Detail gezeigt, wobei Figur 2 eine Vorderansicht des Kompressionstextils im geöffneten Zustand und Figur 3 eine Rückansicht des Kompressionstextils zeigt. Das Kompressionstextil umfasst einen sich entlang einer Längsrichtung L erstreckenden Kompressionsabschnitt 1 aus einem Kompressionsgestrick. Der Kompressionsabschnitt 1 verläuft in Längsrichtung L zwischen einem proximalen Randabschnitt 1c und einem distalen Randabschnitt 1d und weist zwei seitliche Randabschnitte 1a, 1b auf, wie aus Figur 3 ersichtlich. Der Kompressionsabschnitt 1 ist zum Anlegen an einem Unterschenkel und dem Fuß F eines Patienten geformt und weist hierfür im proximalen Bereich einen Wadenabschnitt auf, der quer zur Längsrichtung L etwas breiter ausgebildet ist als im distalen Bereich. An den Wadenabschnitt schließt sich in distaler Richtung ein Fersenabschnitt und ein Sohlenabschnitt an. Der Fersenabschnitt und der Sohlenabschnitt sind dabei so geformt, dass der Kompressionsabschnitt 1 in seinem distalen Bereich am Fuß F des Patienten angelegt werden kann, wobei der Sohlenabschnitt an der Fußsohle und der Fersenabschnitt im Fersenbereich zu liegen kommen (wie auch aus Figur 1 ersichtlich).

Der Kompressionsabschnitt 1 ist aus einem Kompressionsgestrick und bevorzugt aus einem Zweizug-Kompressionsgestrick gefertigt. Das Kompressionsgestrick weist dabei zumindest ein Grundgestrick sowie zumindest einen darin eingelegten oder eingebundenen elastischen Schussfaden auf, der sich quer zur Längsrichtung L erstreckt. Das Grundgestrick weist zumindest einen ersten, maschenbildend verstrickten und zumindest im Wesentlichen unelastischen oder nur geringfügig elastischen Gestrickfaden aus textilem Material auf. Der elastischen Schussfaden ist dabei in das Grundgestrick eingelegt oder unter Ausbildung von Bindungsstellen eingebunden. Das Grundgestrick kann neben dem ersten Gestrickfaden auch noch einen zweiten Gestrickfaden oder weitere Gestrickfäden enthalten, die wie der erste Gestrickfaden ebenfalls maschenbildend in dem Grundgestrick verstrickt sind und elastisch oder auch unelastisch sein können. Bei Verwendung eines maschenbildend in dem Grundgestrick verstrickten elastischen Gestrickfadens ist das Kompressionsgestrick sowohl in Längsrichtung L als auch senkrecht dazu (in Querrichtung) dehnbar. Die Kompressionswirkung des Kompressionsgestricks wird dabei in erster Linie durch die Elastizität des in Querrichtung verlaufenden elastischen Schussfadens hervorgerufen. Zur Herstellung des Kompressionsgestricks können Polyamid-, Elasthan-, Baumwoll-, Elastodien- und Viskose-Fäden oder Microfasern, auch in Kombination miteinander, insbesondere auch in Form von umwundenen bzw. umwickelten Kernfäden eingesetzt werden.

Der elastische Schussfaden ist bevorzugt so in das Grundgestrick eingelegt oder eingebunden, dass der von dem Kompressionsgestrick auf die Körperextremität ausgeübte Druck einen Druckgradienten erzeugt, der zweckmäßig von proximal nach distal stetig zunimmt. Dadurch wird der venöse Blutfluss sowie der Lymphfluss durch das Kompressionsgestrick gefördert.

An den beiden seitlichen Randabschnitten 1a, 1b des Kompressionsabschnitts 1 schließt sich jeweils in Umfangsrichtung (quer zur Längsrichtung L) ein Laschenband 12, 14 an, wobei die Laschenbänder 12, 14 an den seitlichen Randabschnitten 1a, 1b des Kompressionsabschnitts 1, bspw. durch Vernähen entlang einer Längsnaht 9, befestigt sind (Figur 3). An dem ersten Laschenband 12 ist eine erste Gruppe von Befestigungslaschen 2 angeordnet und an dem zweiten Laschenband 14 ist eine zweite Gruppe von Befestigungslaschen 4 angeordnet. Die erste Gruppe von Befestigungslaschen 2 umfasst in dem zeichnerisch in Figur 2 dargestellten Ausführungsbeispiel vier Befestigungslaschen 2a, 2b, 2c, 2d, die in Längsrichtung L nebeneinander liegend und in geringem Abstand zueinander angeordnet sind. In entsprechender Weise umfasst auch die zweite Gruppe von Befestigungslaschen 4 in dem dargestellten Ausführungsbeispiel der Figur 2 vier Befestigungslaschen 4a, 4b, 4c, 4d, die ebenfalls in Längsrichtung L nebeneinander und in geringem Abstand zueinander angeordnet sind. Benachbarte Befestigungslaschen sind sowohl in der ersten Gruppe von Befestigungslaschen 2 als auch in der zweiten Gruppe von Befestigungslaschen 4 jeweils durch Einschnitte 10 voneinander getrennt. Durch diese Einschnitte 10, die in dem jeweiligen Laschenband 12, 14 eingebracht sind, können die Befestigungslaschen der ersten Gruppe von Befestigungslaschen 2 und die Befestigungslaschen der zweiten Gruppe von Befestigungslaschen 4 in den jeweiligen Laschenband 12, 14 ausgebildet werden.

Soweit im Folgenden auf die Befestigungslaschen 2a bis 2d der ersten Gruppe von Befestigungslaschen 2 bzw. auf die Befestigungslaschen 4a bis 4d der zweiten Gruppe von Befestigungslaschen 4 Bezug genommen wird, erfolgt dies durch die Bezugszeichen 2 (für die erste Gruppe von Befestigungslaschen) bzw. 4 (für die zweite Gruppe von Befestigungslaschen).

Durch die Ausbildung der Befestigungslaschen der ersten Gruppe 2 durch die Einschnitte 10 im ersten Laschenband 12 liegen benachbarte Befestigungslaschen der ersten Gruppe 2 direkt nebeneinander, d.h. zwischen der Unterkante der oberen (proximalen) Befestigungslasche 2a und der Oberkante der sich distal daran anschließenden Befestigungslasche 2b liegt lediglich ein geringer Zwischenraum, der durch einen Einschnitt 10 gebildet ist. Entsprechend liegt zwischen der Oberkante der unteren (distalen) Befestigungslasche 2c und der Unterkante der sich in proximaler Richtung daran anschließenden Befestigungslasche 2b lediglich ein geringer Zwischenraum, der ebenfalls durch einen Einschnitt 10 gebildet ist. Entsprechendes gilt für die Befestigungslaschen der zweiten Gruppe 4. Wenn die Befestigungslaschen der ersten Gruppe 2 und die Befestigungslaschen der zweiten Gruppe 4 an der Körperextremität angelegt und befestigt sind, fluchten die Einschnitte 10 der ersten Gruppe 2 und die Einschnitte 10 der zweiten Gruppe 4 miteinander oder sie liegen übereinander, d.h. ein Einschnitt 10 der ersten Gruppe 2 liegt über einem Einschnitt 10 der zweiten Gruppe 4.

Die Befestigungslaschen der ersten Gruppe 2 und die Befestigungslaschen der zweiten Gruppe 4 sind zweckmäßig jeweils aus demselben Material gefertigt wie die Laschenbänder 12, 14, an denen die Befestigungslaschen angeordnet sind. Bevorzugt sind die Befestigungslaschen einstückig am jeweiligen Laschenband 12, 14 angeformt. Bei dem Material, aus dem die Laschenbänder 12, 14 und die daran angeordneten Befestigungslaschen 2, 4 gefertigt sind, handelt es sich um ein elastisches Textilmaterial und bevorzugt um ein elastisches Gewebe. Dabei kann es sich beispielsweise um ein Textilgewebe aus Polyamid und Elasthan, insbesondere mit 87 % Polyamid und 13 % Elasthan, bezogen auf das Gewicht des Textilgewebes, handeln. Bevorzugt weist das elastische Textilmaterial eine materialspezifische Dehnungscharakteristik auf, die eine maximale Dehnung des Materials im Bereich von 15 % bis 50 % der ungedehnten Länge ermöglicht. Besonders bevorzugt ist die maximale Dehnung im Bereich von 20 % bis 40 % der ungedehnten Länge. Bei dem elastischen Textilmaterial, aus dem sowohl die Laschenbänder 12, 14 als auch die daran zweckmäßig einstückig angeformten Befestigungslaschen 2, 4 hergestellt sind, kann es sich auch um andere elastische Textilmaterialen handeln, wie z.B. Lycra, Nylon oder Mischungen davon. Bei dem elastischen Textilmaterial kann es sich um Gewebe oder auch um Vliese oder Gestricke handeln.

Die Befestigungslaschen der ersten Gruppe 2 verfügen jeweils über erste Befestigungsmittel 3. Bei den ersten Befestigungsmitteln 3 handelt es sich bevorzugt um Klettverschlüsse und insbesondere um die Hakenteile (Widerhäkchen) von Klettverschlüssen. Diese können entweder an den Befestigungslaschen der ersten Gruppe 2 befestigt sein, insbesondere an den freien Enden der Befestigungslaschen der ersten Gruppe 2. Die Befestigungsmittel 3 können jedoch auch abnehmbar an den Befestigungslaschen der ersten Gruppe 2 angeordnet sein. Mittels der Befestigungsmittel 3 können die Befestigungslaschen der ersten Gruppe 2 an dem gegenüberliegenden Randabschnitt 1b des Kompressionsabschnitts 1 oder an den gegenüberliegenden Befestigungslaschen der zweiten Gruppe 4 befestigt werden, um das Kompressionstextil an einer Körperextremität zu fixieren. Nachfolgend wird das Anlegen eines Kompressionstextils an einem Unterschenkel eines Patienten unter Bezugnahme auf Figur 2 erläutert:
Zunächst wird das Kompressionstextil am Fuß F und am Unterschenkel des Patienten platziert, wie aus Figur 2 ersichtlich. Dabei kommt der Sohlenabschnitt des Kompressionsabschnitts 1 im Bereich der Fußsohle, der Fersenabschnitt des Kompressionsabschnitts 1 im Bereich der Ferse und der Wadenabschnitt des Kompressionsabschnitts 1 im Bereich der Wade zu liegen. Zur Festlegung des Kompressionstextils am Fuß und Unterschenkel wird zunächst die distale Befestigungslasche 4d der zweiten Gruppe 4 manuell in Querrichtung (quer zur Längsrichtung L) gezogen, und dabei unter Zugspannung gesetzt. Danach wird auch die distale Befestigungslasche 2d der ersten Gruppe 2 in Querrichtung gezogen und über die distale Befestigungslasche 4d der zweiten Gruppe 2 gelegt sowie mittels der Befestigungsmittel 3, an der gegenüberliegenden distalen Befestigungslasche 4d fixiert. Wenn es sich bei den Befestigungsmitteln 3 um abnehmbare Befestigungsmittel handelt, beispielsweise um abnehmbare Hakenteile eines Klettverschlusses, werden diese bspw. in einem ersten Abschnitt auf die Außenseite der distalen Befestigungslasche 2d der ersten Gruppe 2 und in einem weiteren Abschnitt auf die Außenseite der darunterliegenden distalen Befestigungslasche 4d der zweiten Gruppe 4 gelegt und angedrückt. Dadurch werden die distalen Befestigungslaschen 2d, 4d fest miteinander verbunden, wobei die beiden distalen Befestigungslaschen 2d, 4d unter Zugspannung gehalten werden.

In entsprechender Weise werden dann die in Längsrichtung L sich nach proximal anschließenden Befestigungslaschen 2c, 4c; 2b, 4b und 2a, 4a um den Unterschenkel geschlungen und mittels der ersten Befestigungsmittel 3 fixiert. Dabei werden die Befestigungslaschen 2, 4 jeweils unter Zugspannung gehalten. Durch die Zugspannung der Befestigungslaschen 2, 4 und wegen der Dehnbarkeit des Kompressionsabschnitts 1 in Querrichtung wird bei angelegtem Kompressionstextil ein Kompressionsdruck auf die darunterliegende Körperextremität ausgeübt.

Zweckmäßig weist das Kompressionsgestrick des Kompressionsabschnitts 1 dabei einen Kompressionsgradienten auf, mit einem von proximal nach distal zunehmenden Kompressionsdruck. Hierfür ist das Kompressionsgestrick des Kompressionsabschnitts so ausgebildet, dass es in Querrichtung eine von proximal nach distal abnehmende Dehnbarkeit aufweist. Wenn das Kompressionsgestrick des Kompressionsabschnitts 1 einen entsprechenden Kompressionsgradienten aufweist, erzeugt das an einer Körperextremität angelegte Kompressionstextil einen von proximal nach distal zunehmenden Kompressionsdruck auf die darunter liegende Körperextremität, wodurch der venöse Blutfluss unterstützt und beschleunigt wird. Zweckmäßig erzeugt das Kompressionsgestrick des Kompressionsabschnitts dabei einen Kompressionsdruck auf das darunter liegende Körpergewebe des Patienten aus, der im Bereich einer der Kompressionsklassen I bis IV liegt, die in der DIN 58133 bzw. dem RAL-Gütezeichen RAL-GZ 387/1 definiert sind.

Ergänzend zu den ersten Befestigungsmitteln 3, die fest oder abnehmbar an den Befestigungslaschen der ersten Gruppe 2 angeordnet sind, können auch die Befestigungslaschen der zweiten Gruppe 4 mit zweiten Befestigungsmitteln 5 ausgestattet sein. Bei den zweiten Befestigungsmitteln 5 handelt es sich zweckmäßig ebenfalls um Klettverschlüsse, insbesondere um die Hakenteile von Klettverschlüssen. Diese können, wie in Figur 2 angedeutet, beispielsweise auf der Außenseite der Befestigungslaschen der zweiten Gruppe 4 und insbesondere im Bereich der freien Enden der Befestigungslaschen angeordnet sein. Durch diese Anordnung von zweiten Befestigungsmitteln 5 an den Befestigungslaschen der zweiten Gruppe 4 wird das Anlegen des Kompressionstextils an einer Körperextremität erleichtert und eine genauere Positionierung und eine exakte Einstellung einer gewünschten Zugspannung der Befestigungslaschen ermöglicht. Bei Verwendung von zweiten Befestigungsmitteln 5 an den Befestigungslaschen der zweiten Gruppe 4 können die gegenüberliegenden Befestigungslaschen der ersten Gruppe 2 auf die Befestigungslaschen der zweiten Gruppe 4 aufgelegt und dort mittels der zweiten Befestigungsmittel 5 fixiert werden.

Weiterhin kann, wie ebenfalls aus Figur 2 ersichtlich, an der Innenseite der proximalen Befestigungslaschen 2a, 4a sowie an der Innenseite des proximalen Randabschnitts 1c des Kompressionsabschnitts 1 ein Haftband 6 angeordnet sein. Dieses Haftband 6, das beispielsweise aus einem Gummi- oder Silikonmaterial gefertigt sein kann, verhindert ein Abrutschen des Kompressionstextils an der Körperextremität B.

Die aus dem Unterschenkel bzw. dem Fuß F herausragenden Knochenstifte 13 des externen Fixateurs 11 können beim Anlegen des Kompressionstextils zwischen benachbarten Befestigungslaschen 2, 4 und insbesondere durch die Einschnitte 10, durch welche benachbarte Befestigungslaschen voneinander getrennt sind, hindurchgeführt werden. Dies wird insbesondere dadurch ermöglicht, dass die Einschnitte 10 der ersten Gruppe 2 und die Einschnitte 10 der zweiten Gruppe 4 bei angelegtem Kompressionstextil übereinander liegen bzw. miteinander fluchten. Das Kompressionstextil kann daher trotz der Anordnung des externen Fixateurs 11 an der Körperextremität zur postoperativen Kompressionsbehandlung angelegt werden. Dabei kann entsprechend den individuellen Behandlungsbedürfnissen ein gewünschter Kompressionsdruck eingestellt werden. Unterschiedliche Kompressionsdrücke können dabei durch Variation der beim Anlegen des Kompressionstextils auf die Befestigungslaschen 2, 4 aufgebrachten Zugspannungen und deren Fixierung mittels der ersten Befestigungsmittel 3 und ggf. der zweiten Befestigungsmittel 5 realisiert werden.

Bei dem zeichnerisch dargestellten Ausführungsbeispiel ist im Wadenbereich des Kompressionstextils eine Ausnehmung 20 im Kompressionsabschnitt 1 vorgesehen, welche durch das elastische Textilmaterial des angrenzenden Laschenbands 12 ausgefüllt ist. In diesem Bereich der Ausnehmung 20 ist ein in Querrichtung gesehen bis etwa zur Mitte der Wade reichender Einschnitt 10' in dem Textilmaterial vorgesehen, durch den ein Knochenstift 13' durchgeführt werden kann, der im Bereich der Wade aus dem Unterschenkel herausragt, wie in Figur 1 gezeigt.

Der Kompressionabschnitt 1 kann, wie aus Figur 3 ersichtlich, aus zwei Teilen 1', 1" zusammengesetzt sein, die entlang einer Längsnaht 8 miteinander vernäht sind. Es ist jedoch auch möglich, das Kompressionsgestrick des Kompressionabschnitts 1 nahtlos aus einem Stück zu stricken, was in Bezug auf den Tragekomfort vorteilhaft ist.

Bei dem Kompressionstextil kann eine individuelle Anpassung und Maßanfertigung des Kompressionstextils an den verwendeten Fixateur 11 erfolgen. Hierfür kann bspw. die Anzahl der Befestigungslaschen der ersten Gruppe 2 und der zweiten Gruppe 4 an die Ausgestaltung des Fixateurs 11 und insbesondere an die Anzahl und die Anordnung der Knochenstifte 13 angepasst werden. So zeigt das Ausführungsbeispiel der Figur 1 ein Kompressionstextil mit fünf Befestigungslaschen 2a - 2e und in Figur 2 ist ein Ausführungsbeispiel eines Kompressionstextils mit vier Befestigungslaschen 2a - 2d gezeigt. Die Anzahl der Befestigungslaschen der ersten Gruppe 2 und der zweiten Gruppe 4 ist dabei jeweils gleich. In entsprechender Weise können auch die Längen der Einschnitte 10 in Querrichtung (bzw. bei an einer Körperextremität angelegtem Kompressionstextil in Umfangsrichtung der Körperextremität) an die Lage der Knochenstifte 13 angepasst werden.

Es ist jedoch auch möglich nur eine erste Gruppe 2 von Befestigungslaschen vorzusehen, wobei deren Befestigungslaschen dann an dem gegenüberliegenden seitlichen Randabschnitt 1b des Kompressionsabschnitts 1 mittels der ersten Befestigungsmittel 3 fixiert werden. Um zu vermeiden, dass die als Klettverschlüsse ausgebildeten ersten Befestigungsmittel 3 auf der Außenseite des gegen mechanische Einflüsse empfindlichen Kompressionsgestricks befestigt werden müssen, kann dabei auch vorgesehen sein, an dem gegenüberliegenden Randabschnitt 1b des Kompressionsabschnitts 1 ein in Längsrichtung L verlaufendes Klettband aus einem zweckmäßig elastischen Textilmaterial mit an der Oberfläche ausgebildeten Klettschlaufen zu befestigen, in welche die an den Befestigungslaschen 2 angeordneten Hakenteile der Klettverschlüsse zur Ausbildung einer Klettverbindung einhaken können.

Die Anwendung der Erfindung ist nicht auf das beschriebene Ausführungsbeispiel beschränkt. So kann das Kompressionstextil in Verbindung mit einem externen Fixateur auch zur postoperativen Kompressionsbehandlung eines Oberschenkels oder auch im Unter- oder Oberarmbereich eingesetzt werden.

## Patentansprüche

1. System zur postoperativen Kompressionsbehandlung einer Körperextremität bestehend aus einem Kompressionstextil und einem externen Fixateur (11) mit einer Mehrzahl von Knochenstiften (13), die von außen in einem Knochen der Körperextremität befestigbar sind, wobei das Kompressionstextil umfasst:
- einen sich entlang einer Längsrichtung (L) erstreckenden Kompressionsabschnitt (1) aus einem Kompressionsgestrick, mit einem ersten und einem zweiten seitlichen Randabschnitt (1a, 1b),
- eine erste Gruppe von Befestigungslaschen (2) mit mehreren Befestigungslaschen (2a, 2b, 2c, 2d, 2e) aus einem elastischen Textilmaterial, welche an dem ersten seitlichen Randabschnitt (1a) des Kompressionsabschnitts (1) angeordnet sind und jeweils über erste Befestigungsmittel (3) verfügen, über welche die jeweilige Befestigungslasche (2a, 2b, 2c, 2d, 2e) an dem gegenüberliegenden Randabschnitt (1a, 1b) des Kompressionsabschnitts (1) und/oder an gegenüberliegenden Befestigungslaschen (4a, 4b, 4c, 4d, 4e) einer zweiten Gruppe von Befestigungslaschen (4) befestigbar ist, wenn das Kompressionstextil um die Körperextremität geschlungen ist,
- wobei benachbarte Befestigungslaschen (2a, 2b, 2c, 2d, 2e) der ersten Gruppe von Befestigungslaschen (2) jeweils durch Einschnitte (10) voneinander getrennt sind.

2. System nach Anspruch 1, **gekennzeichnet durch** eine zweite Gruppe von Befestigungslaschen (4) mit einer Mehrzahl von Befestigungslaschen (4a, 4b, 4c, 4d, 4e), welche an dem zweiten seitlichen Randabschnitt (1b) des Kompressionsabschnitts (1) angeordnet und aus einem elastischen Textilmaterial gefertigt sind, wobei benachbarte Befestigungslaschen (4a, 4b, 4c, 4d, 4e) der zweiten Gruppe von Befestigungslaschen (4) jeweils durch Einschnitte (10) voneinander getrennt sind.

3. System nach Anspruch 2, **dadurch gekennzeichnet, dass** die Befestigungslaschen (4a, 4b, 4c, 4d, 4e) der zweiten Gruppe von Befestigungslaschen (4) jeweils über zweite Befestigungsmittel (5) verfügen, über welche die jeweilige Befestigungslasche (4a, 4b, 4c) an einer gegenüberliegenden Befestigungslasche (2a, 2b, 2c, 2d, 2e) der ersten Gruppe von Befestigungslaschen (2) befestigbar ist.

4. System nach einem der voranstehenden Ansprüche, wobei Knochenstifte des externen Fixateurs durch die Einschnitte (10) der ersten Gruppe von Befestigungslaschen (2) und/oder durch die Einschnitte (10) der zweiten Gruppe von Befestigungslaschen (4) durchgreifen, wenn das Kompressionstextil bei befestigtem Fixateur an der Körperextremität angelegt ist.

5. System nach einem der voranstehenden Ansprüche, **dadurch gekennzeichnet, dass** die Befestigungslaschen (2a, 2b, 2c, 2d, 2e) der ersten Gruppe von Befestigungslaschen (2) und die optional vorhandenen Befestigungslaschen (4a, 4b, 4c, 4d, 4e) der zweiten Gruppe von Befestigungslaschen (4) bei angelegtem Kompressionstextil unter Zug befestigt sind, so dass auf die Körperextremität ein Kompressionsdruck ausgeübt wird, wobei der von dem Kompressionstextil auf die Körperextremität ausgeübte Kompressionsdruck durch die Positionierung der Befestigung der Befestigungslaschen (2a, 2b, 2c, 2d, 2e) der ersten Gruppe von Befestigungslaschen (2) und/oder der Befestigungslaschen (4a, 4b, 4c, 4d, 4e) der zweiten Gruppe von Befestigungslaschen (4) quer zur Längsrichtung (L) einstellbar ist.

6. System nach einem der voranstehenden Ansprüche, **dadurch gekennzeichnet, dass** die ersten Befestigungsmittel (3) und/oder die zweiten Befestigungsmittel (5) Klettverschlüsse und/oder Hakenverschlüsse und/oder Druckknöpfe umfassen.

7. System nach einem der voranstehenden Ansprüche, **dadurch gekennzeichnet, dass** die erste Gruppe von Befestigungslaschen (2) mindestens zwei Befestigungslaschen (2a, 2b, 2c, 2d, 2e) umfasst und/oder dass die zweite Gruppe von Befestigungslaschen (4) mindestens zwei Befestigungslaschen (4a, 4b, 4c, 4d, 4e) umfasst.

8. System nach einem der voranstehenden Ansprüche, **dadurch gekennzeichnet, dass** die Befestigungslaschen (2a, 2b, 2c, 2d, 2e) der ersten Gruppe von Befestigungslaschen (2) in Längsrichtung (L) im Abstand zueinander und nebeneinander liegend angeordnet sind und/oder dass die Befestigungslaschen (4a, 4b, 4c, 4d, 4e) der zweiten Gruppe von Befestigungslaschen (4) in Längsrichtung (L) im Abstand zueinander und nebeneinander liegend angeordnet sind.

9. System nach einem der voranstehenden Ansprüche, **dadurch gekennzeichnet, dass** die Befestigungslaschen (2a, 2b, 2c, 2d, 2e) der ersten Gruppe von Befestigungslaschen (2) an einem ersten Laschenband (12) angeordnet sind, welches mit einem seitlichen Randabschnitt (1a) des Kompressionsabschnitts (1) verbunden ist, wobei sich die Befestigungslaschen (2a, 2b, 2c, 2d, 2e) von einem Endabschnitt (12a) des ersten Laschenbands (12) erstrecken und/oder dass die Befestigungslaschen (4a, 4b, 4c, 4d, 4e) der zweiten Gruppe von Befestigungslaschen (4) an einem zweiten Laschenband (14) angeordnet sind, welches mit einem seitlichen Randabschnitt (1b) des Kompressionsabschnitts (1) verbunden ist, wobei sich die Befestigungslaschen von einem Endabschnitt (14a) des zweiten Laschenbands (14) erstrecken.

10. System nach einem der voranstehenden Ansprüche, **dadurch gekennzeichnet, dass** das Kompressionsgestrick ein Flachgestrick ist, insbesondere ein Zweizug-Flachgestrick, welches ein Grundgestrick mit wenigstens einem darin eingelegten oder eingebundenen elastischen Schussfaden umfasst.

11. System nach einem der voranstehenden Ansprüche, wobei das Kompressionsgestrick so gefertigt ist, dass der vom Kompressionsgestrick auf die Körperextremität ausgeübte Druck bei angelegtem Kompressionstextil von proximal nach distal zunimmt.

12. System nach einem der voranstehenden Ansprüche, **dadurch gekennzeichnet, dass** die Befestigungslaschen (2a, 2b, 2c, 2d, 2e) der ersten Gruppe von Befestigungslaschen (2) und/oder die Befestigungslaschen (4a, 4b 4c, 4d, 4e) der zweiten Gruppe von Befestigungslaschen (4) aus einem elastischen Gewebe, insbesondere aus einem elastischen Gewebe mit einer Dehnbarkeit im Bereich von 10% bis 50% und bevorzugt im Bereich von 20% bis 40% gebildet sind.

13. System nach einem der voranstehenden Ansprüche, **dadurch gekennzeichnet, dass** die erste Gruppe von Befestigungslaschen (2) eine erste distale Befestigungslasche (2d, 2e) umfasst und dass die zweite Gruppe von Befestigungslaschen (4) eine zweite distale Befestigungslasche (4d, 4e) umfasst, wobei die erste und die zweite distale Befestigungslasche (2d, 2e, 4d, 4e) beim Anlegen des Kompressionstextils jeweils um einen Fuß eines Patienten geschlungen und dort mittels der Befestigungsmittel (3, 5) fixiert werden.

14. System nach einem der voranstehenden Ansprüche, **dadurch gekennzeichnet, dass** das Kompressionsgestrick aus einem Grundgestrick aus einem elastischen oder unelastischen Gestrickfaden und wenigstens einem in dem Grundgestrick eingelegten oder eingebundenen elastischen Schussfaden besteht.

## Claims

1. System for post-operative compression treatment of a body extremity consisting of a compression textile and an external fixer (11) having a plurality of bone pins (13) which are attachable from the outside in a bone of the body extremity, wherein the compression textile comprises:
- a compression section (1) extending along a longitudinal direction (L) made of a compression knitted fabric, having a first and a second lateral edge section (1a, 1b),
- a first group of attachment straps (2) having several attachment straps (2a, 2b, 2c, 2d, 2e) made of an elastic textile material which are arranged on the first lateral edge section (1a) of the compression section (1) and have respectively first attachment means (3), via which the respective attachment strap (2a, 2b, 2c, 2d, 2e) is attachable to the opposite edge section (1a, 1b) of the compression section (1) and/or to opposite attachment straps (4a, 4b, 4c, 4d, 4e) of a second group of attachment straps (4) if the compression textile is wrapped around the body extremity.
- wherein adjoining attachment straps (2a, 2b, 2c, 2d, 2e) of the first group of attachment straps (2) are separated from one another respectively by indentations (10).

2. System according to claim 1, **characterised by** a second group of attachment straps (4) having a plurality of attachment straps (4a, 4b, 4c, 4d, 4e) which are arranged on the second lateral edge section (1b) of the compression section (1) and manufactured from an elastic textile material, wherein adjoining attachment straps (4a, 4b, 4c, 4d, 4e) of the second group of attachment straps (4) are separated from one another respectively by indentations (10).

3. System according to claim 2, **characterised in that** the attachment straps (4a, 4b, 4c, 4d, 4e) of the second group of attachment straps (4) have respectively second attachment means (5), via which the respective attachment strap (4a, 4b, 4c) is attachable to an opposite attachment strap (2a, 2b, 2c, 2d, 2e) of the first group of attachment straps (2).

4. System according to one of the preceding claims, wherein bone pins of the external fixer penetrate through the indentations (10) of the first group of attachment straps (2) and/or through the indentations (10) of the second group of attachment straps (4) if the compression textile is applied to the body extremity when the fixer is attached.

5. System according to one of the preceding claims, **characterised in that** the attachment straps (2a, 2b, 2c, 2d, 2e) of the first group of attachment straps (2) and the optionally present attachment straps (4a, 4b, 4c, 4d, 4e) of the second group of attachment straps (4) are attached under tension when the compression textile is applied so that a compression pressure is exerted on the body extremity, wherein the compression pressure exerted by the compression textile on the body extremity is adjustable by positioning of attachment of the attachment straps (2a, 2b, 2c, 2d, 2e) of the first group of attachment straps (2) and/or of the attachment straps (4a, 4b, 4c, 4d, 4e) of the second group of attachment straps (4) transversely to the longitudinal direction (L).

6. System according to one of the preceding claims, **characterised in that** the first attachment means (3) and/or the second attachment means (5) comprise Velcro closures and/or hook closures and/or press-studs.

7. System according to one of the preceding claims, **characterised in that** the first group of attachment straps (2) comprises at least two attachment straps (2a, 2b, 2c, 2d, 2e) and/or **in that** the second group of attachment straps (4) comprises at least two attachment straps (4a, 4b, 4c, 4d, 4e).

8. System according to one of the preceding claims, **characterised in that** the attachment straps (2a, 2b, 2c, 2d, 2e) of the first group of attachment straps (2) are arranged in the longitudinal direction (L) at a distance from one another and lying next to one another and/or **in that** the attachment straps (4a, 4b, 4c, 4d, 4e) of the second group of attachment straps (4) are arranged in the longitudinal direction (L) at a distance from one another and lying next to one another.

9. System according to one of the preceding claims, **characterised in that** the attachment straps (2a, 2b, 2c, 2d, 2e) of the first group of attachment straps (2) are arranged on a first strap band (12) which is connected to a lateral edge section (1a) of the compression section (1), wherein the attachment straps (2a, 2b, 2c, 2d, 2e) extend from an end section (12a) of the first strap band (12) and/or **in that** the attachment straps (4a, 4b, 4c, 4d, 4e) of the second group of attachment straps (4) are arranged on a second strap band (14) which is connected to a lateral edge section (1b) of the compression section (1), wherein the attachment straps extend from an end section (14a) of the second strap band (14).

10. System according to one of the preceding claims, **characterised in that** the compression knitted fabric is a flat knitted fabric, in particular a double-stretch flat knitted fabric, which comprises a base knitted fabric having at least one elastic weft thread inserted or bound therein.

11. System according to one of the preceding claims, wherein the compression knitted fabric is manufactured so that the pressure exerted by the compression knitted fabric on the body extremity increases proximally to distally when the compression textile is applied.

12. System according to one of the preceding claims, **characterised in that** the attachment straps (2a, 2b, 2c, 2d, 2e) of the first group of attachment straps (2) and/or the attachment straps (4a, 4b, 4c, 4d, 4e) of the second group of attachment straps (4) are formed from an elastic fabric, in particular from an elastic fabric having a stretchability in the range from 10% to 50% and preferably in the range from 20% to 40%.

13. System according to one of the preceding claims, **characterised in that** the first group of attachment straps (2) comprises a first distal attachment strap (2d, 2e) and **in that** the second group of attachment straps (4) comprises a second distal attachment strap (4d, 4e), wherein the first and the second distal attachment strap (2d, 2e, 4d, 4e) when applying the compression textile are wrapped respectively around a foot of a patient and fixed there by means of the attachment means (3, 5).

14. System according to one of the preceding claims, **characterised in that** the compression knitted fabric consists of a base knitted fabric made of an elastic or non-elastic knitted fabric thread and at least one elastic weft thread inserted or bound in the base knitted fabric.

## Revendications

1. Système pour le traitement par compression post-opératoire d'une extrémité du corps, constitué d'un textile de compression et d'un fixateur externe (11) pourvu d'une pluralité de broches osseuses (13), qui peuvent être fixées de l'extérieur dans un os de l'extrémité du corps, dans lequel le textile de compression comprend :
- une partie de compression (1), composée d'un tricot de compression, s'étendant le long d'une direction longitudinale (L), avec une première et une deuxième partie de bord latéral (1a, 1b),
- un premier groupe de languettes de fixation (2) avec plusieurs languettes de fixation (2a, 2b, 2c, 2d, 2e) composées d'un matériau textile élastique, lesquelles sont disposées sur la première partie de bord latéral (1a) de la partie de compression (1) et disposent respectivement de premiers moyens de fixation (3), par l'intermédiaire desquels la languette de fixation (2a, 2b, 2c, 2d, 2e) respective peut être fixée sur la partie de bord latéral (1a, 1b) opposée de la partie de compression (1) et/ou sur des languettes de fixation (4a, 4b, 4c, 4d, 4e) opposées d'un deuxième groupe de languettes de fixation (4), lorsque le textile de compression est enroulé autour de l'extrémité du corps,
- dans lequel des languettes de fixation (2a, 2b, 2c, 2d, 2e) voisines du premier groupe de languettes de fixation (2) sont séparées les unes des autres par des entailles (10).

2. Système selon la revendication 1, **caractérisé par** un deuxième groupe de languettes de fixation (4) avec une pluralité de languettes de fixation (4a, 4b, 4c, 4d, 4e), lesquelles sont disposées sur la deuxième partie de bord latéral (1b) de la partie de compression (1) et fabriquées à partir d'un matériau textile élastique, dans lequel des languettes de fixation (4a, 4b, 4c, 4d, 4e) voisines du deuxième groupe de languettes de fixation (4) sont séparées les unes des autres respectivement par des entailles (10).

3. Système selon la revendication 2, **caractérisé en ce que** les languettes de fixation (4a, 4b, 4c, 4d, 4e) du deuxième groupe de languettes de fixation (4) disposent respectivement de deuxièmes moyens de fixation (5), par l'intermédiaire desquels la languette de fixation (4a, 4b, 4c) respective peut être fixée sur une languette de fixation (2a, 2b, 2c, 2d, 2e) opposée du premier groupe de languettes de fixation (2).

4. Système selon l'une quelconque des revendications précédentes, dans lequel des broches osseuses du fixateur externe traversent les entailles (10) du premier groupe de languettes de fixation (2) et/ou les entailles (10) du deuxième groupe de languettes de fixation (4), lorsque le textile de compression est placé sur l'extrémité du corps lorsque le fixateur est fixé.

5. Système selon l'une quelconque des revendications précédentes, **caractérisé en ce que** les languettes de fixation (2a, 2b, 2c, 2d, 2e) du premier groupe de languettes de fixation (2) et les languettes de fixation (4a, 4b, 4c, 4d, 4e) éventuellement présentes du deuxième groupe de languettes de fixation (4) sont fixées sous tension lorsque le textile de compression est placé, de sorte qu'une pression de compression est exercée sur l'extrémité du corps, dans lequel la pression de compression exercée par le textile de compression sur l'extrémité du corps peut être réglée par le positionnement de la fixation des languettes de fixation (2a, 2b, 2c, 2d, 2e) du premier groupe de languettes de fixation (2) et/ou des languettes de fixation (4a, 4b, 4c, 4d, 4e) du deuxième groupe de languettes de fixation (4) transversalement à la direction longitudinale (L).

6. Système selon l'une quelconque des revendications précédentes, **caractérisé en ce que** les premiers moyens de fixation (3) et/ou les deuxièmes moyens de fixation (5) comprennent des fermetures à boucles et crochets et/ou fermetures à crochets et/ou boutons-pression.

7. Système selon l'une quelconque des revendications précédentes, **caractérisé en ce que** le premier groupe de languettes de fixation (2) comprend au moins deux languettes de fixation (2a, 2b, 2c, 2d, 2e) et/ou que le deuxième groupe de languettes de fixation (4) comprend au moins deux languettes de fixation (4a, 4b, 4c, 4d, 4e).

8. Système selon l'une quelconque des revendications précédentes, **caractérisé en ce que** les languettes de fixation (2a, 2b, 2c, 2d, 2e) du premier groupe de languettes de fixation (2) sont disposées de manière à se situer les unes à côté des autres et à distance les unes des autres dans la direction longitudinale (L) et/ou que les languettes de fixation (4a, 4b, 4c, 4d, 4e) du deuxième groupe de languettes de fixation (4) sont disposées les unes à côté des autres et à distance les unes des autres dans la direction longitudinale (L).

9. Système selon l'une quelconque des revendications précédentes, **caractérisé en ce que** les languettes de fixation (2a, 2b, 2c, 2d, 2e) du premier groupe de languettes de fixation (2) sont disposées sur une première bande de languettes (12), laquelle est reliée à une partie de bord latéral (1a) de la partie de compression (1), dans lequel les languettes de fixation (2a, 2b, 2c, 2d, 2e) s'étendent à partir d'une partie d'extrémité (12a) de la première bande de languettes (12) et/ou que les languettes de fixation (4a, 4b, 4c, 4d, 4e) du deuxième groupe de languettes de fixation (4) sont disposées sur une deuxième bande de languettes (14), laquelle est reliée à une partie de bord latéral (1b) de la partie de compression (1), dans lequel les languettes de fixation s'étendent à partir d'une partie d'extrémité (14a) de la deuxième bande de languettes (14).

10. Système selon l'une quelconque des revendications précédentes, **caractérisé en ce que** le tricot de compression est un tricot plat, en particulier un tricot plat deux tensions, lequel comprend un tricot de base avec au moins un fil de trame élastique inséré ou intégré dans celui-ci.

11. Système selon l'une quelconque des revendications précédentes, dans lequel le tricot de compression est fabriqué de sorte que la pression exercée par le tricot de compression sur l'extrémité du corps augmente de proximal à distal lorsque le textile de compression est placé.

12. Système selon l'une quelconque des revendications précédentes, **caractérisé en ce que** les languettes de fixation (2a, 2b, 2c, 2d, 2e) du premier groupe de languettes de fixation (2) et/ou les languettes de fixation (4a, 4b, 4c, 4d, 4e) du deuxième groupe de languettes de fixation (4) sont formées d'un tissu élastique, en particulier d'un tissu élastique avec une extensibilité dans la plage de 10 % à 50 % et de préférence dans la plage de 20 % à 40 %.

13. Système selon l'une quelconque des revendications précédentes, **caractérisé en ce que** le premier groupe de languettes de fixation (2) comprend une première languette de fixation (2d, 2e) distale et que le deuxième groupe de languettes de fixation (4) comprend une deuxième languette de fixation (4d, 4e) distale, dans lequel la première et la deuxième languette de fixation (2d, 2e, 4d, 4e) distale, lorsque le textile de compression est placé, sont enroulées respectivement autour d'un pied d'un patient et fixées à cet endroit au moyen des moyens de fixation (3, 5).

14. Système selon l'une quelconque des revendications précédentes, **caractérisé en ce que** le tricot de compression est constitué d'un tricot de base composé d'un fil de tricot élastique ou non élastique et d'au moins un fil de trame élastique inséré ou intégré dans le tricot de base.
